# EUROPÄISCHE PATENTSCHRIFT

(11) **EP 2 670 452 B1**
(45) Veröffentlichungstag und Bekanntmachung des Hinweises auf die Patenterteilung: **23.08.2017**
(21) Anmeldenummer: 12702965.0
(22) Anmeldetag: 30.01.2012
(51) Int. Cl.: A61M 1/14, E05C 9/00, A61M 1/16

(54) **MEDIZINISCHES GERÄT**
MEDICAL APPLIANCE
APPAREIL MÉDICAL

(30) Priorität: 03.02.2011 DE 102011010249; 03.02.2011 US 201161457220 P
(43) Veröffentlichungstag der Anmeldung: 11.12.2013
(73) Patentinhaber: Fresenius Medical Care Deutschland GmbH, 61352 Bad Homburg (DE)
(72) Erfinder: FÖRGER, Jens, 35789 Laubuseschbach (DE); OESTERREICH, Stefan, 61267 Neu-Anspach (DE)
(74) Vertreter: Laufhütte, Dieter
(86) Internationale Anmeldenummer: PCT/EP2012/000400
(87) Internationale Veröffentlichungsnummer: WO 2012/104056

(56) Entgegenhaltungen:
- EP-A1- 1 707 720
- US-A- 1 567 662
- US-A1- 2005 095 152
- US-A1- 2009 107 902

## Beschreibung

Die Erfindung betrifft ein medizinisches Gerät, insbesondere eine Vorrichtung zur extrakorporalen Blutbehandlung, nach dem Oberbegriff des Anspruchs 1.

Ein solches medizinisches Gerät ist aus der Druckschrift US 2005/095152 A1 bekannt.

Medizinische Geräte, wie beispielsweise Vorrichtungen zur extrakorporalen Blutbehandlung, weisen üblicherweise ein Gehäuse auf, das sehr häufig aus Kostengründen aus einer einfachen Blechkonstruktion gefertigt ist. Das Gehäuse könnte auch eine tragende Kunststoffkonstruktion sein. Denkbar ist insbesondere, daß das Gehäuse eine tragende Blechkonstruktion aufweist, die mit Kunststoffteilen umbaut wird. In dem Gehäuse ist in der Regel zumindest eine Tür vorgesehen, die eine Revisionsöffnung für den Servicetechniker freigibt. Üblicherweise ist an der Tür ein entsprechender Griff zum Öffnen der Tür angeordnet.

Es sind Vorrichtungen zur extrakorporalen Blutbehandlung als sogenannte Akut-Dialysemaschinen bekannt, die zum Einsatz in Intensivstationen dienen und hierzu auf Rollen verfahrbar ausgestaltet sind. Um diese ungefähr 85 kg schwere Akut-Dialysemaschine zu bewegen, wird sie üblicherweise an dem Türgriff gehalten. Somit kommt dem Türgriff eine Doppelfunktion zu. Einerseits dient er dem Servicetechniker zum Öffnen und Schließen der Tür und andererseits dem Anwender der Dialysemaschine in der Klinik als Griff zum Schieben der gesamten Dialysemaschine. Während des Schiebens werden von dem Griffmechanismus Kräfte auf das Gehäuse übertragen.

In manchen Anwendungsfällen ist die Tür als gesamte Gehäusefront ausgebildet und mit einer umlaufenden elastischen Dichtung versehen, die beim Schließen an die Zarge des feststehenden Gehäuses gedrückt wird. Dabei darf die Dichtung weder zu stark noch zu schwach angedrückt werden. Auch müssen Fertigungstoleranzen ausgeglichen werden, die aufgrund der Verwendung der abgekanteten günstigen Blechkonstruktion unvermeidlich sind.

Trotz dieser Fertigungstoleranzen soll einerseits die Tür ohne Verzug umlaufend dichtend anliegen und andererseits soll der Griff beim Schieben der Dialysemaschine kein spürbares Spiel aufweisen. Bei der Verwendung bekannter Türgriffe wird die Dichtung beim Schieben der Dialysemaschine zusätzlich verformt. Ein spürbares Nachgeben des Griffs während des Anschiebens durch den Anwender verleiht der gesamten Dialysemaschine eine negative Qualitätsanmutung, die es zu vermeiden gilt.

Aufgabe der vorliegenden Erfindung ist es demnach, ein medizinisches Gerät an die Hand zu geben, welches einen Türgriff aufweist, der sich trotz eines einfachen Aufbaus massiv anfühlen und beim Schieben des medizinischen Gerätes nicht nachgeben soll. Gleichzeitig soll über den Griff die Tür des medizinischen Gerätes geöffnet und geschlossen werden können.

Erfindungsgemäß wird diese Aufgabe durch ein medizinisches Gerät mit den Merkmalen des Anspruchs 1 gelöst. Demnach weist das medizinische Gerät ein eine Tür umfassendes Gehäuse auf und einen an der Tür angeordneten Griffmechanismus, über den die Tür mit dem Gehäuse über einen Verriegelungsmechanismus verriegelbar ist. An dem Griffmechanismus ist in seinem in das Gehäuse hineinragenden Teil ein Teil des Verriegelungsmechanismus ausgebildet, der mit dem anderen gehäuseseitigen Teil des Verriegelungsmechanismus zusammenwirkt. Dabei durchringt der Griffmechanismus die Tür längsverschieblich. Vorgesehen ist, daß am Griffmechanismus in seinem in das Gehäuse hineinragenden Teil ein Teil des Verriegelungsmechanismus ausgebildet ist und daß wenigstens eine Formschlußeinheit vorgesehen ist, die wenigstens ein Formschlußmittel und wenigstens ein Formschlußmittelgegenstück aufweist, wobei beim Verriegeln und/oder Verschließen der Tür das wenigstens eine Formschlußmittel in das wenigstens eine Formschlußmittelgegenstück zumindest teilweise eingreift.

Dadurch ergibt sich der Vorteil, daß eine definierte Positionierung der Tür gegenüber dem Gehäuse erreicht werden kann. Insbesondere ist dabei vorteilhaft, daß ein korrektes Schließen hierdurch einfach und sicher gewährleistet werden kann, wobei zugleich ein einfacher Aufbau möglich ist.

Das Gehäuse kann aus einer einfachen Blechkonstruktion gefertigt ist. Das Gehäuse könnte auch eine tragende Kunststoffkonstruktion sein. Vorteilhaft denkbar ist insbesondere, daß das Gehäuse eine tragende Blechkonstruktion aufweist, die mit Kunststoffteilen umbaut wird. Das medizinische Gerät kann in vorteilhafter Ausgestaltung eine Dialysemaschine, z. B. eine Akutdialysemaschine sein.

Vorteilhafte Ausgestaltungen der Erfindung ergeben sich aus den sich an den Hauptanspruch anschließenden Unteransprüchen.

Es kann vorgesehen sein, daß wenigstens ein Formschlußmittel und/oder wenigstens ein Formschlußmittelgegenstück am Griffmechanismus angeordnet und/oder angeformt und/oder befestigt ist.

Denkbar ist ferner, daß wenigstens ein Formschlußmittel und/oder wenigstens ein Formschlußmittelgegenstück gehäuseseitig angeordnet und/oder angeformt und/oder befestigt ist.

Vorteilhaft möglich ist insbesondere, daß gehäuseseitig und griffmechanismusseitig sowohl wenigstens ein Formschlußmittel und wenigstens ein Formschlußmittelgegenstück vorgesehen sind. Genausogut ist aber auch möglich, daß gehäuseseitig das Formschlußmittel und griffmechanismusseitig das Formschlußmittelgegenstück vorgesehen ist. Denkbar ist aber auch als weitere genauso vorteilhafte Möglichkeit, daß griffmechanismusseitig das Formschlußmittel und gehäuseseitig das Formschlußmittelgegenstück vorgesehen ist.

Darüber hinaus kann vorgesehen sein, daß das wenigstens eine Formschlußmittel und/oder wenigstens eine Formschlußmittelgegenstück elastisch gelagert ist.

Möglich ist weiter, daß die Formschlußeinheit einen Kegelzapfen als Formschlußmittel und eine kegelförmige Aufnahme als Formschlußmittelgegenstück aufweist.

Es ist denkbar, daß der Griffmechanismus beim Verschließen über mindestens eine kegelförmige Aufnahme in mindestens einen gehäuseseitig elastisch gelagerten Kegelzapfen eingreift und/oder daß der Griffmechanismus beim Verschließen über mindestens einen griffmechanismusseitig elastisch gelagerten Kegelzapfen in mindestens eine gehäuseseitige kegelförmige Aufnahme eingreift. Beim Schließen der Tür mittels des Verriegelungsmechanismus wird so vorteilhaft die mindestens eine kegelförmige Aufnahme in den mindestens einen gehäuseseitig und/oder griffmechanismusseitig elastisch gelagerten Kegelzapfen derart vorgespannt, daß die üblichen vom Griff aufgenommenen Schubkräfte beim Schieben des medizinischen Geräts auf das Gehäuse übertragen werden können, ohne daß sich der Griff gegenüber dem Gehäuse verlagert. Gleichzeitig kann der mindestens eine elastisch gelagerte Kegelzapfen in allen Richtungen die fertigungsbedingten Toleranzen vorteilhaft ausgleichen.

Es ist ferner möglich, daß wenigstens ein Federmittel vorgesehen ist, mittels dessen das wenigstens eine Formschlußmittel vorspannbar ist.

Vorteilhafterweise kann vorgesehen sein, daß das Federmittel eine Druckfeder, eine Schraubenfeder und/oder eine Tellerfeder ist und/oder umfaßt und/oder daß das Federmittel zumindest teilweise durch elastische Bereiche des Gehäuses ausgebildet ist.

Denkbar ist insbesondere, daß der mindestens eine Kegelzapfen über mindestens eine Tellerfeder vorgespannt ist. Durch den Einsatz der Tellerfedern können die Kegelzapfen dreidimensional elastisch am Gehäuse, genauer an der Gehäusezarge, vorteilhaft gelagert werden.

Dabei können die Tellerfedern um einen gewünschten Betrag vorgespannt werden, um nach dem Schließen der Tür und dem elastischen Ausgleich eventueller Toleranzen dennoch eine hinreichend starre Kraftübertragung vom Griff auf das Gehäuse zu ermöglichen.

Gemäß einer weiteren bevorzugten Ausgestaltung der Erfindung sind mehrere Kegelzapfen in mindestens zwei Ebenen im Gehäuse angeordnet. Vorzugsweise sind hier vier Kegelzapfen in zwei unterschiedlichen Ebenen der Gehäusezargen vorhanden. Eine alternative Ausführung kann aber auch darin liegen, daß in einer Ebene zwei Kegelzapfen und in einer zweiten Ebene ein einziger Kegelzapfen vorhanden ist.

Der Verriegelungsmechanismus kann einen schwenkbaren Haken aufweisen, der mit einem Zugbolzen in bekannter Weise zur Verriegelung und Freigabe der Tür zusammenwirkt. Dabei kann der schwenkbare Haken in üblicher Weise mit einem Riegel versehen sein, über den der Verriegelungsmechanismus geöffnet werden kann.

Gemäß einer Ausführungsvariante weist der Griffmechanismus mindestens eine Zugfeder, die beim Schließen der Tür spannbar ist, auf. Aufgrund der Federkraft der mindestens einen Zugfeder erfolgt eine definierte Verpressung einer zwischen der Tür und dem Gehäuse, das heißt genauer der Gehäusezarge, angeordneten Dichtung. Die Anpreßkraft ist gemäß einer weiteren bevorzugten Ausgestaltung in Abhängigkeit von der verwendeten Dichtung ausgelegt. Insoweit wird durch diese Ausführungsvariante zusätzlich noch eine gleichmäßige Beaufschlagung der Dichtung sichergestellt.

Es ist weiter denkbar, daß zumindest ein Teil der Gehäusefront als Tür ausgebildet ist.

Gemäß einer vorteilhaften Ausführungsvariante ist die gesamte Gehäusefront als Tür ausgebildet. Dabei kann die Tür um ihre untere Kante drehbar gelagert sein, wie dies beispielsweise von einer Backofentür bekannt ist, während der Griffmechanismus an der gegenüberliegenden Seite der Tür, also nahe der Oberkante angeordnet ist.

Weitere Merkmale, Einzelheiten und Vorteile der Erfindung werden anhand eines in der Zeichnung dargestellten Ausführungsbeispiels näher erläutert. Es zeigen:
- Figur 1:: eine Schnittdarstellung durch einen Teil des medizinischen Gerätes nach einer Ausführungsvariante der vorliegenden Erfindung,
- Figur 2:: eine weitere Schnittdarstellung des Details gemäß Figur 1 in geöffneter Stellung,
- Figur 3:: die Detaildarstellung des medizinischen Geräts gemäß Figur 2 in geschlossener aber noch nicht verriegelter Position,
- Figur 4:: eine perspektivische, teilweise aufgeschnittene Darstellung des medizinischen Geräts entsprechend Figur 2 in geöffneter Stellung,
- Figur 5:: eine Darstellung gemäß Figur 4 in geschlossener Stellung und
- Figur 6:: einen Detailschnitt durch einen weiteren Teil des medizinischen Gerätes.

In den Figuren ist lediglich der Griffbereich bzw. Verschlußmechanismus einer ein Gehäuse eines hier nicht näher dargestellten medizinischen Geräts verschließenden Tür 12 dargestellt. Bei dem medizinischen Gerät kann es sich vorteilhaft, aber nicht ausschließlich um eine Akut-Dialysemaschine handeln, die üblicherweise auf Rollen gelagert ist, um sie in einer Intensivstation zu dem jeweiligen Intensivbett zum Einsatz fahren zu können.

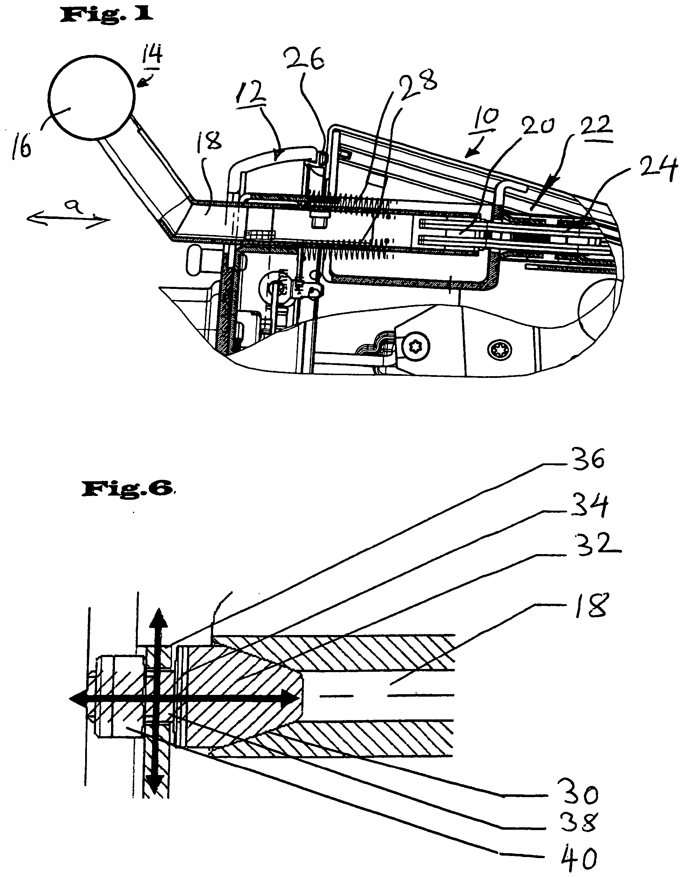

Hierzu wird die gesamte Vorrichtung am Griff 14 gefaßt. Der Griff 14 weist eine Griffstange 16 auf, die am freien aus der Tür 12 herausragenden Ende eines Griffstegs 18 angesetzt ist. Der Griffsteg 18 durchsetzt die Tür 12 und ist verschiebbar in ihr gelagert, wie insbesondere aus der Figur 1 zu ersehen ist. Im Griffsteg 18 ist am in das Gehäuse 10 hineinragenden freien Ende ein Zugbolzen 20 eingelassen, der Teil einer Verriegelungseinrichtung 22 ist.

Diese Verriegelungseinrichtung 22 weist im wesentlichen einen schwenkbaren Haken 24 auf, der mit dem Zugbolzen 20 zusammenwirkt und nach entsprechendem Verriegeln des Zugbolzens 20 die Tür sichert. Die Konstruktion der Verriegelungseinrichtung 22 ist konventionell aufgebaut und als Spannverschluß für eine Stumpfstoßverriegelung käuflich zu erwerben. Sie ermöglicht ein automatisches Einschnappen des Hakens 24 am Zugbolzen beim Schließen der Tür 12 sowie eine Freigabe des Zugbolzens 20 durch den Haken 24 nach entsprechendem Öffnen durch eine hier nicht näher dargestellte Handhabe.

Sowohl das gesamte Gehäuse 10 wie auch die Tür 12 sind aus einer gekanteten Blechkonstruktion hergestellt. Am oberen Rand der Tür 12 ist zum Gehäuse 10 hin gerichtet eine umlaufende Dichtung 26 vorgesehen.

Die Dichtung soll möglichst gleichmäßig an der umgekanteten Auflagekante bzw. Zarge des Gehäuses 10 anliegen.

Um hier ein sicheres rundum geschlossenes Anliegen der Dichtung 26 im Bereich zwischen der Tür 12 und dem Gehäuse 10 sicherzustellen ist der in der Tür 12 in Doppelpfeilrichtung A gemäß Figur 1 verschieblich gelagerte Griff 14 über Federn 28 vorspannbar. So wird der Griff zum Schließen der Tür 12 gegen die Federkraft der Federn 28 in die Tür hineingedrückt bis der Haken 24 den Zugbolzen 20 sichert. Die Federkraft der Federn 28 ist dabei so gewählt, daß ein möglichst gleichmäßiger Druck auf die Tür 12 ausgeübt wird, so daß die Dichtung 26 sicher an dem umlaufenden Rand des Gehäuses 10 aufliegt und ihre Dichtfunktion erfüllt.

Im hier dargestellten Ausführungsbeispiel sind vier Zugfedern 28 vorgesehen. Beim Schließen der Tür trifft also zunächst die Tür 12 auf die umlaufende Zarge des Gehäuses 10, während sich der Griff 14 weiter durch die Durchführung hindurch in seine Verriegelungsposition bewegt und dabei die Zugfedern 28 spannt. Die Anpresskraft der Zugfedern ist zur definierten Verpressung der Tür mit der Gehäusezarge jeweils in Abhängigkeit von der verwendeten Dichtung auszulegen. Bei einer mittelweichen Dichtung kann eine Anpresskraft zwischen 150 und 200 N gewählt werden. Hierdurch können auch relativ große Fertigungstoleranzen, die durch das Fertigen der Blechteile bedingt sind, ausgeglichen werden.

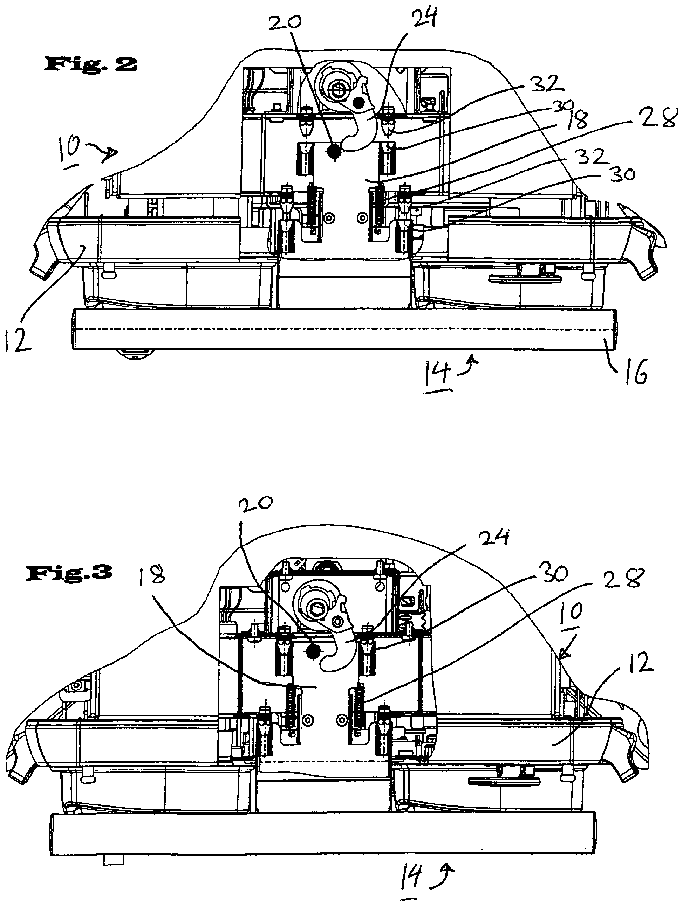

Aus den Figuren 2, 3, 4 und 5 ist weiterhin ersichtlich, daß am Griffsteg 18 kegelförmige Aufnahmen 30 ausgenommen sind. Diese wirken beim Verschließen der Tür 12 mit entsprechenden Kegelzapfen 32 zusammen. In der hier gezeigten Ausführungsform umfaßt die Formschlußeinheit die kegelförmigen Aufnahmen 30 und die entsprechenden Kegelzapfen 32. Die Formschlußmittel sind dabei durch die Kegelzapfen 32 ausgebildet und die Formschlußmittelgegenstücke sind dabei durch die kegelförmigen Aufnahmen 30 ausgebildet.

Anhand der Figur 6 kann die Wirkungsweise der ineinandergreifenden kegelförmigen Aufnahmen 30 und des Kegelbolzens 32 erläutert werden. Der Kegelbolzen 32 weist an seiner Unterseite ein Schraubgewinde 38 auf, über das er durch den Rahmen 36 des Gehäuses 10 steckbar und mit einer Mutter 14 festlegbar ist. Auf der gegenüberliegenden Seite des Rahmens 36 sind auf den Gewindebolzen 38 Telerfedern aufgeschoben, die über die Mutter 40 vorgespannt werden können. Wie aus der Figur 6 weiter zu ersehen ist, ist um den Gewindebolzen 38 in der den Rahmen 36 durchgreifenden Bohrung ein ausreichendes radiales Spiel vorhanden. Aufgrund der vorgespannten Tellerfedern, über die sich der Kegelbolzen 32 über dem Rahmen 36 des Gehäuses 10 abstützt und dem radialen Spiel im Bereich der Bohrung ist eine dreidimensionale elastische Lagerung gewährleistet, wie dies durch die Bewegungspfeile in Figur 6 angedeutet ist.

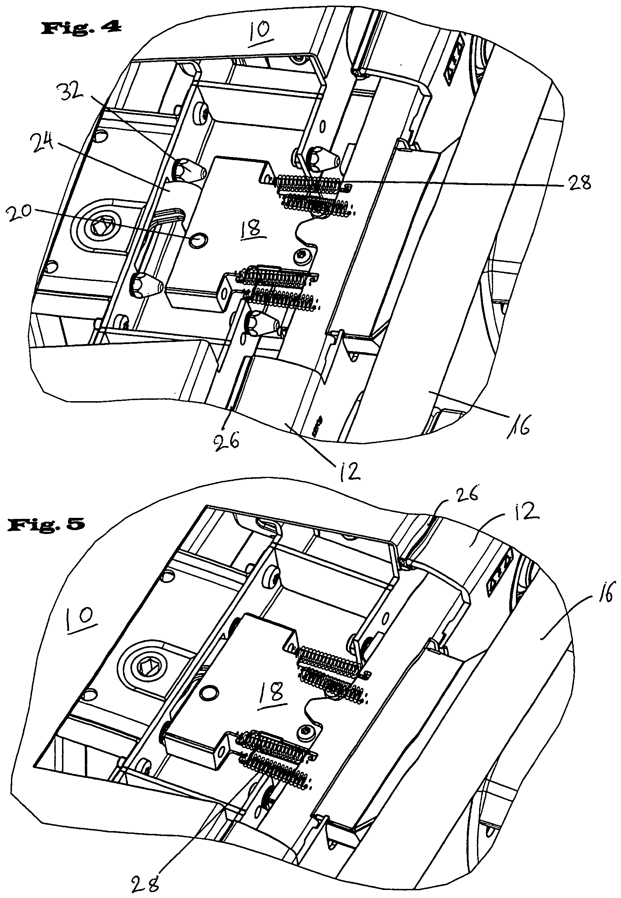

Wie nun aus den Figuren 2, 3, 4 und 5 zu sehen, sind entsprechende Kegelbolzen 32 und kegelförmige Aufnahmen 30 an mehreren Stellen des Gehäuses 10 bzw. des Griffs 14 vorgesehen. So sind in dieser Ausführungsvariante zwei Kegelbolzen 32 in den zurückspringenden Teil des Gehäuses angeordnet, in welchem der Haken 24 drehbar gelagert ist. Wie aus den Figuren deutlich zu ersehen ist, sind in dem entsprechenden Bereich des Griffstegs 18 die zugehörigen kegelförmigen Aufnahmen ausgebildet, wobei in den Figuren 2 und 4 die Aufnahmen 30 noch nicht auf den Kegelzapfen aufsitzen. In den Figuren 3 und 5 sind die kegelförmigen Aufnahmen 30 dagegen bereits mit den gehäuseseitig elastisch gelagerten Kegelzapfen gekoppelt. Dabei werden auch zwei Kegelzapfen 32 mit weiteren am Griffsteg 18 angeordneten kegelförmigen Aufnahmen 30 gekoppelt, die im äußeren Rahmenbereich des Gehäuses 10 und damit in einer anderen Ebene als die zuvor beschriebenen Kegelzapfen 32 angeordnet sind.

Die Federsteifigkeit der bei den Kegelbolzen verwendeten Tellerfedern 34 ist so ausgelegt, daß die üblichen Schubkräfte beim Schieben des medizinischen Geräts, das heißt im vorliegenden Beispiel der Dialysemaschine, nicht zu spürbaren elastischen Verformungen der Tellerfedern führen kann. Hierdurch ergibt sich das haptische Gefühl eines "massiven" und starren Griffs 14 und einer unmittelbaren Kraftübertragung der Schubkräfte auf das medizinische Gerät. Weiterhin gleichen die dreidimensional elastisch gelagerten Kegelzapfen 32 in allen Richtungen die fertigungsbedingten Toleranzen aus. Neben den in dieser Ausführungsvariante dargestellten vier Kegelzapfen 32 könnte auch eine Ausführungsform nur aus drei oder zwei Kegelzapfen, die auf zwei Ebenen angeordnet sind, bestehen. Auch eine Ausführungsform mit einem einzigen Kegelzapfen kann bei entsprechender Dimensionierung bereits den erfindungsgemäßen Effekt erbringen.

## Patentansprüche

1. Medizinisches Gerät, insbesondere eine Vorrichtung zur extrakorporalen Blutbehandlung, mit einem eine Tür (12) aufweisenden Gehäuse (10) und mit einem an der Tür (12) angeordneten Griffmechanismus, über den die Tür (12) mit dem Gehäuse (10) über einen Verriegelungsmechanismus (22) verriegelbar ist, wobei am Griffmechanismus in seinem in das Gehäuse (10) hineinragenden Teil ein Teil des Verriegelungsmechanismus (22) ausgebildet ist,
**dadurch gekennzeichnet,**
**daß** der Griffmechanismus längsverschieblich die Tür (12) durchdringt und wenigstens eine Formschlußeinheit vorgesehen ist, die wenigstens ein Formschlußmittel und wenigstens ein Formschlußmittelgegenstück aufweist, wobei beim Verriegeln und/oder Verschließen der Tür (12) das wenigstens eine Formschlußmittel in das wenigstens eine Formschlußmittelgegenstück zumindest teilweise eingreift.

2. Medizinisches Gerät nach Anspruch 1, **dadurch gekennzeichnet, daß** wenigstens ein Formschlußmittel und/oder wenigstens ein Formschlußmittelgegenstück am Griffmechanismus angeordnet und/oder angeformt und/oder befestigt ist.

3. Medizinisches Gerät nach Anspruch 1 oder 2, **dadurch gekennzeichnet, daß** wenigstens ein Formschlußmittel und/oder wenigstens ein Formschlußmittelgegenstück gehäuseseitig angeordnet und/oder angeformt und/oder befestigt ist.

4. Medizinisches Gerät nach einem der vorhergehenden Ansprüche, **dadurch gekennzeichnet, daß** das wenigstens eine Formschlußmittel und/oder wenigstens eine Formschlußmittelgegenstück elastisch gelagert ist.

5. Medizinisches Gerät nach einem der vorhergehenden Ansprüche, **dadurch gekennzeichnet, daß** die Formschlußeinheit einen Kegelzapfen (32) als Formschlußmittel und eine kegelförmige Aufnahme (30) als Formschlußmittelgegenstück aufweist.

6. Medizinisches Gerät nach Anspruch 5, **dadurch gekennzeichnet, daß** der Griffmechanismus beim Verschließen über mindestens eine kegelförmige Aufnahme (30) in mindestens einen gehäuseseitig elastisch gelagerten Kegelzapfen (32) eingreift und/oder daß der Griffmechanismus beim Verschließen über mindestens einen griffmechanismusseitig elastisch gelagerten Kegelzapfen (32) in mindestens eine gehäuseseitige kegelförmige Aufnahme (30) eingreift.

7. Medizinisches Gerät nach einem der vorhergehenden Ansprüche, **dadurch gekennzeichnet, daß** wenigstens ein Federmittel vorgesehen ist, mittels dessen das wenigstens eine Formschlußmittel vorspannbar ist.

8. Medizinisches Gerät nach Anspruch 7, **dadurch gekennzeichnet, daß** das Federmittel eine Druckfeder, eine Schraubenfeder und/oder eine Tellerfeder ist und/oder umfaßt und/oder daß das Federmittel zumindest teilweise durch elastische Bereiche des Gehäuses (10) ausgebildet ist.

9. Medizinisches Gerät nach Anspruch 8, **dadurch gekennzeichnet, daß** der mindestens eine Kegelzapfen (32) über mindestens eine Tellerfeder vorgespannt ist.

10. Medizinisches Gerät nach einem der Ansprüche 5 bis 9, **dadurch gekennzeichnet, daß** mehrere Kegelzapfen (32) in mindestens zwei Ebenen im Gehäuse (10) angeordnet sind.

11. Medizinisches Gerät nach einem der vorhergehenden Ansprüche, **dadurch gekennzeichnet, daß** der Verriegelungsmechanismus (22) einen schwenkbaren Haken (24) aufweist, der mit einem Zugbolzen (20) zur Verriegelung und Freigabe der Tür (12) zusammenwirkt.

12. Medizinisches Gerät nach einem der vorhergehenden Ansprüche, **dadurch gekennzeichnet, daß** der Griffmechanismus fest mit der Tür (12) verbunden ist.

13. Medizinisches Gerät nach einem der vorhergehenden Ansprüche, **dadurch gekennzeichnet, daß** der Griffmechanismus mindestens eine Zugfeder (28) aufweist, die beim Schließen der Tür (12) spannbar ist.

14. Medizinisches Gerät nach Anspruch 13, **dadurch gekennzeichnet, daß** zwischen der Tür (12) und dem Gehäuse (10) eine Dichtung (26) angeordnet ist und daß die Anpreßkraft der mindestens einen Zugfeder (28) derart gewählt ist, daß die Dichtung (26) bei geschlossener Tür (12) weitgehend gleichmäßig zusammengepreßt ist.

15. Medizinisches Gerät nach einem der vorhergehenden Ansprüche, **dadurch gekennzeichnet, daß** zumindest ein Teil der Gehäusefront als Tür (12) ausgebildet ist.

16. Medizinisches Gerät nach Anspruch 15, **dadurch gekennzeichnet, daß** die gesamte Gehäusefront als Tür (12) ausgebildet ist.

17. Medizinisches Gerät nach einem der vorhergehenden Ansprüche, **dadurch gekennzeichnet, daß** die Tür (12) um ihre untere Kante drehbar gelagert ist, während der Griffmechanismus an der gegenüberliegenden Seite der Tür (12) ausgebildet ist.

## Claims

1. A medical appliance, in particular an apparatus for the extracorporeal blood treatment, with a housing (10) including a door (12) and with a handle mechanism arranged at the door (12), by means of which the door (12) can be locked with the housing (10) via a locking mechanism (22), wherein at the handle mechanism, in its part protruding into the housing (10), a part of the locking mechanism (22) is formed,
**characterized in**
**that** the handle mechanism longitudinally shiftably penetrates the door (12) and at least one form-fit unit is provided, which includes at least one form-fit means and at least one form-fit means counterpart, wherein on locking and/or closing of the door (12) the at least one form-fit means at least partly engages in the at least one form-fit means counterpart.

2. The medical appliance according to claim 1, **characterized in that** at least one form-fit means and/or at least one form-fit means counterpart is arranged and/or integrally molded and/or attached at the handle mechanism.

3. The medical appliance according to claim 1 or 2, **characterized in that** at least one form-fit means and/or at least one form-fit means counterpart is arranged and/or integrally integrally molded and/or attached on the side of the housing.

4. The medical appliance according to any of the preceding claims, **characterized in that** the at least one form-fit means and/or at least one form-fit means counterpart is elastically mounted.

5. The medical appliance according to any of the preceding claims, **characterized in that** the form-fit unit includes a tapered spigot (32) as form-fit means and a conical receptacle (30) as form-fit means counterpart.

6. The medical appliance according to claim 5, **characterized in that** on closing the handle mechanism engages in at least one tapered spigot (32) elastically mounted on the side of the housing via at least one conical receptacle (30) and/or that on closing the handle mechanism engages in at least one conical receptacle (30) on the side of the housing via at least one tapered spigot (32) elastically mounted on the side of the handle mechanism.

7. The medical appliance according to any of the preceding claims, **characterized in that** at least one spring means is provided, by means of which the at least one form-fit means can be pretensioned.

8. The medical appliance according to claim 7, **characterized in that** the spring means is and/or comprises a compression spring, a coil spring and/or a disk spring and/or that the spring means is at least partly formed by elastic regions of the housing (10).

9. The medical appliance according to claim 8, **characterized in that** the at least one tapered spigot (32) is pretensioned via at least one disk spring.

10. The medical appliance according to any of claims 5 to 9, **characterized in that** a plurality of tapered spigots (32) are arranged in at least two planes in the housing (10).

11. The medical appliance according to any of the preceding claims, **characterized in that** the locking mechanism (22) includes a pivotable hook (24) which cooperates with a tension bolt (20) for locking and unlocking the door (12).

12. The medical appliance according to any of the preceding claims, **characterized in that** the handle mechanism is firmly connected with the door (12).

13. The medical appliance according to any of the preceding claims, **characterized in that** the handle mechanism includes at least one tension spring (28) which can be tensioned on closing the door (12).

14. The medical appliance according to claim 13, **characterized in that** between the door (12) and the housing (10) a seal (26) is arranged and that the pressing force of the at least one tension spring (28) is chosen such that with the door (12) closed the seal (26) is compressed largely uniformly.

15. The medical appliance according to any of the preceding claims, **characterized in that** at least a part of the housing front is formed as door (12).

16. The medical appliance according to claim 15, **characterized in that** the entire housing front is formed as door (12).

17. The medical appliance according to any of the preceding claims, **characterized in that** the door (12) is rotatably mounted about its lower edge, whereas the handle mechanism is formed on the opposite side of the door (12).

## Revendications

1. Appareil médical, en particulier un dispositif de traitement extracorporel du sang, avec un boîtier (10) comprenant une porte (12) et avec un mécanisme de poignée disposé sur la porte (12), par lequel la porte (12) peut être verrouillée avec le boîtier (10) par un mécanisme de verrouillage (22), une partie du mécanisme de verrouillage (22) étant formée sur le mécanisme de poignée dans sa partie faisant saillie dans le boîtier (10),
**caractérisé en ce que**
le mécanisme de poignée traverse la porte (12) de manière à coulisser longitudinalement et au moins une unité de liaison de forme est prévue, qui comprend au moins un moyen de liaison de forme et au moins une contre-pièce du moyen de liaison de forme, dans lequel en verrouillant et/ou fermant la porte (12) le au moins un moyen de liaison de forme s'engage au moins partiellement dans la au moins une contre-pièce du moyen de liaison de forme.

2. Appareil médical selon la revendication 1, **caractérisé en ce qu'**au moins un moyen de liaison de forme et/ou au moins une contre-pièce du moyen de liaison de forme est disposé(e) et/ou intégralement moulé(e) et/ou fixé(e) sur le mécanisme de poignée.

3. Appareil médical selon les revendications 1 ou 2, **caractérisé en ce qu'**au moins un moyen de liaison de forme et/ou au moins une contre-pièce du moyen de liaison de forme est disposé(e) et/ou intégralement moulé(e) et/ou fixé(e) du côté du boîtier.

4. Appareil médical selon l'une quelconque des revendications précédentes, **caractérisé en ce que** le au moins un moyen de liaison de forme et/ou la au moins une contre-pièce du moyen de liaison de forme est monté(e) élastiquement.

5. Appareil médical selon l'une quelconque des revendications précédentes, **caractérisé en ce que** l'unité de liaison de forme comprend un pivot conique (32) comme moyen de liaison de forme et un évidement conique (30) comme contre-pièce du moyen de liaison de forme.

6. Appareil médical selon la revendication 5, **caractérisé en ce que** lors de la fermeture par au moins un évidement conique (30) le mécanisme de poignée s'engage dans au moins un pivot conique (32) monté élastiquement du côté du boîtier et/ou que lors de la fermeture par au moins un pivot conique (32) monté élastiquement du côté du mécanisme de poignée le mécanisme de poignée s'engage dans au moins un évidement conique (30) du côté du boîtier.

7. Appareil médical selon l'une quelconque des revendications précédentes, **caractérisé en ce qu'**au moins un moyen à ressort est prévu, au moyen duquel le au moins un moyen de liaison de forme peut être précontraint.

8. Appareil médical selon la revendication 7, **caractérisé en ce que** le moyen à ressort est et/ou comporte un ressort de compression, un ressort hélicoïdal et/ou un ressort à disques et/ou que le moyen à ressort est au moins partiellement formé par des zones élastiques du boîtier (10).

9. Appareil médical selon la revendication 8, **caractérisé en ce que** le au moins un pivot conique (32) peut être précontraint par au moins un ressort à disques.

10. Appareil médical selon l'une quelconque des revendications 5 à 9, **caractérisé en ce que** plusieurs pivots coniques (32) sont disposés dans au moins deux plans dans le boîtier (10).

11. Appareil médical selon l'une quelconque des revendications précédentes, **caractérisé en ce que** le mécanisme de verrouillage (22) comprend un crochet (24) pivotant, qui coopère avec un boulon tirant (20) pour verrouiller et déverrouiller la porte (12).

12. Appareil médical selon l'une quelconque des revendications précédentes, **caractérisé en ce que** le mécanisme de poignée est fermement relié à la porte (12).

13. Appareil médical selon l'une quelconque des revendications précédentes, **caractérisé en ce que** le mécanisme de poignée comprend au moins un ressort de traction (28), qui peut être contraint en fermant la porte (12).

14. Appareil médical selon la revendication 13, **caractérisé en ce qu'**entre la porte (12) et le boîtier (10) un joint d'étanchéité est disposé et que la force de pression du au moins un ressort de traction (28) est choisie de sorte que le joint d'étanchéité (26) est largement uniformément comprimé lorsque la porte (12) est fermée.

15. Appareil médical selon l'une quelconque des revendications précédentes, **caractérisé en ce qu'**au moins une partie avant du boîtier est formée comme porte (12).

16. Appareil médical selon la revendication 15, **caractérisé en ce que** l'entière partie avant du boîtier est forme comme porte (12).

17. Appareil médical selon l'une quelconque des revendications précédentes, **caractérisé en ce que** la porte (12) est montée rotative autour son bord inférieur, tandis que le mécanisme de poignée est formé du côté opposé de la porte (12).
